# EUROPEAN PATENT APPLICATION

(11) **EP 3 375 414 A1**
(43) Date of publication of application: **19.09.2018**
(21) Application number: 17161595.8
(22) Date of filing: 17.03.2017
(51) Int. Cl.: A61F 5/01, A47C 9/02, A61H 3/00, B25J 9/00

(54) **WEARABLE SITTING POSTURE ASSISTING DEVICE**

(71) Applicant: noonee AG, 8630 Rüti ZH (CH)
(72) Inventor: Gunura, Keith, 8003 Zürich (CH); Motovilova, Olga, 8045 Zürich (CH); Vafi, Daniel, 8006 Zurich (CH)
(74) Representative: Daub, Thomas

(57) **Abstract**

The invention relates to a wearable sitting posture assisting device, comprising at least one body connection unit (10a, 12a; 10b-d), the body connection unit (10a, 12a; 10b-d) featuring at least one support element (14a-d) and at least one body part connector (16a-d) for connecting to at least one thigh (208a) and/or shank (212a) of a person (200a).

It is proposed that the body part connector (16a-d) is movably mounted to the support element (14a-d).

## Description

### State of the Art

The invention relates to a wearable sitting posture assisting device.

A posture assisting device is known from document WO 2015/028373 A1.

The objective of the invention is, in particular, to provide a generic wearable sitting posture assisting device with improved characteristics regarding comfort. Furthermore, an objective of the invention is, in particular, to provide a wearable sitting posture assisting device which is easy to put on and/or take off. Another objective of the invention is, in particular, to allow a person wearing a wearable sitting posture assisting device to at least partly move a body part, in particular a foot, when sitting on the wearable sitting posture assisting device, in particular for improving sitting comfort. The objective is achieved according to the invention by the features of patent claim 1, while advantageous embodiments and further developments of the invention may be gathered from the dependent claims.

### Advantages of the Invention

The invention relates to a wearable sitting posture assisting device, comprising at least one body connection unit, the body connection unit featuring at least one support element and at least one body part connector for connecting to at least one thigh and/or shank of a person.

It is proposed that the body part connector is movably mounted to the support element.

By means of the invention advantageous properties regarding comfort, in particular regarding wearing comfort, can be achieved. Furthermore, a wearable sitting posture assisting device can be provided, which can be put on or taken off easily and/or comfortably. In addition, a high degree of sitting comfort can be achieved. In particular, it is possible for a person to move, in particular to turn and/or to move forward or backward, a foot while sitting on the wearable sitting posture assisting device. Advantageously, it is possible for a person sitting on the wearable sitting posture assisting device to change a sitting position with regard to a positioning of feet without standing up. Furthermore, a person is advantageously enabled to put on a body part connector prior to connecting it to a leg unit, which in particular improves handling and/or comfort. Furthermore, a wearable sitting posture assisting device can be properly worn by persons of different sizes and/or without the need of accurate positioning of a body part connector. Additionally, a high sitting stability can be achieved while enabling a person to unobstructedly stand up while wearing the wearable sitting posture assisting device. Advantageously, a high degree of walking comfort can be achieved. In particular, a person is enabled to freely and/or unobstructedly move while wearing a wearable sitting posture assisting device.

A "wearable sitting posture assisting device" is herein to be understood as a device which is configured for receiving a weight force of a person in a sitting posture or in a partly sitting posture and in particular for transmitting the weight force to a ground. In particular, an angle between a thigh and a shank of at least one leg of the person in the sitting posture is no greater than 130°, preferably no greater than 120° and advantageously no greater than 110° and/or no smaller than 60°, preferably no smaller than 70° and advantageously no smaller than 80°. In particular, the angle between the thigh and the shank of the person is approximately 90° in the sitting posture. In particular, an angle between a thigh and a shank of the person in the partly sitting posture is no greater than 170°, preferably no greater than 160° and advantageously no greater than 150° and/or no smaller than 100°, preferably no smaller than 110° and advantageously no smaller than 120°. In particular, the angle between the thigh and the shank of the person is approximately 130° in the partly sitting posture. It is conceivable that the partly sitting posture is a posture in which the person is leaning forward while partly bending his knees. In particular, a person wearing the wearable sitting posture assisting device is enabled to sit and/or to partly sit and/or to sit down on the wearable sitting posture assisting device, wherein at least a portion of the weight force is counteracted by the wearable sitting posture assisting device, and/or wherein the person counteracts only a fraction of the weight force using his muscles. In particular, the wearable sitting posture assisting device is configured for being worn by the person while the person is standing and/or while the person is walking. Advantageously, the wearable sitting posture assisting device is configured for supporting different sitting postures and/or partly sitting postures, which are in particular characterized by different sitting angles.

The wearable sitting posture assisting device in particular defines a sitting direction. Preferably, the person faces and/or looks in the sitting direction when sitting or partly sitting on the wearable sitting posture assisting device and facing forward. In particular, the sitting direction is oriented parallel to a floor on which the person is standing and/or sitting and/or walking when wearing the wearable sitting posture assisting device. In particular, the wearable sitting posture assisting device defines a walking direction. Preferably, the person faces in the walking direction when walking and/or standing with the wearable sitting posture assisting device and facing forward. In particular, the walking direction is oriented parallel to a floor on which the person is standing and/or sitting and/or walking when wearing the wearable sitting posture assisting device. In particular, the wearable sitting posture assisting device is only designed to receive and transmit the weight force. Preferably, the wearable sitting posture assisting device is not designed to generate a controllable force which is configured to assist a person while walking, standing or lifting some loads. In this context, "configured" is in particular to mean specifically programmed, designed and/or equipped. By an object being configured for a certain function is in particular to be understood that the object implements and/or fulfills said certain function in at least one application state and/or operating state.

Preferably, the wearable sitting posture assisting device comprises at least one leg unit. In particular, the wearable sitting posture assisting device comprises at least one additional leg unit. In particular, the leg unit comprises at least one, preferably one, upper leg and/or at least one, preferably one, lower leg and/or at least one, preferably one, foot unit and/or at least one, preferably one, ground contact unit. Advantageously, the upper leg is connected to the lower leg, in particular via at least one, in particular one, knee joint. Preferably, the foot unit is connected to the lower leg. Advantageously, the ground contact unit is connected to the lower leg and/or to the foot unit. It is conceivable that the ground contact unit is at least partly implemented integrally with the lower leg and/or at least partly implemented integrally with the foot unit. It is also conceivable that the foot unit is at least partly implemented integrally with the lower leg. Preferably, the wearable sitting posture assisting device comprises at least one upper body wearing unit. In particular, the leg unit is connected to the upper body wearing unit, preferably via at least one connection strap. In this context, the term "a first object and a second object being at least partly implemented integrally" is in particular to mean that at least one component of the first object and at least one component of the second object are implemented integrally with each other. "Implemented integrally" is in particular to mean, in this context, connected at least by substance-to-substance bond, e.g., by a welding process, an adhesive bonding, an injection-molding process and/or by another process that is deemed expedient by a person having ordinary skill in the art. Advantageously, "implemented integrally" could in particular mean made of one piece. "Made of one piece" is, in particular, to mean, in this context, manufactured from one single piece, e.g., by production from one single cast and/or by manufacturing in a one-component or multi-component injection-molding process, and advantageously from a single blank.

Preferably, the wearable sitting posture assisting device comprises two leg units. Advantageously, the leg unit and the additional leg unit are implemented identically. It is also conceivable that the leg unit and the additional leg unit are implemented mirror-symmetrically with respect to each other. It is conceivable that the leg unit is configured for being worn on a left leg and the additional leg unit is configured for being worn at a right leg, or vice versa. Advantageously, the leg unit is configured for being worn either on a left leg or on a right leg. Further advantageously, the additional leg unit is configured for being worn on a left leg or on a right leg. Preferably, the additional leg unit is connected to the upper body wearing unit, preferably via at least one connection strap. In particular, the person wearing the wearable sitting posture assisting device wears the leg unit, in particular solely, on a first leg, for instance a left leg or a right leg. In particular, the person wearing the wearable sitting posture assisting device wears the additional leg unit, in particular solely, on a second leg, for instance a right leg or a left leg. Advantageously, the leg unit is arranged on a rear side of the leg on which the leg unit is worn. Further advantageously, the additional leg unit is arranged on a rear side of the leg on which the additional leg unit is worn. In particular, the leg units of the wearable sitting posture assisting device are arranged on respective rear sides of the legs of the person when the person is sitting and/or partly sitting on the wearable sitting posture assisting device and/or standing and/or walking with the wearable sitting posture assisting device. Preferably, the person wearing the wearable sitting posture assisting device wears the upper body wearing unit on his upper body. Advantageously, the upper body wearing unit is implemented as a belt and/or as braces and/or as suspenders. The wearable sitting posture assisting device enables the person wearing it to walk around, to stand, to sit down on the wearable sitting posture assisting device if required or desired and to stand up after sitting or partly sitting on the wearable sitting posture assisting device.

Preferably, the upper leg comprises at least one thigh connection unit for connecting to a thigh of the person. Preferably, the thigh connection unit features at least one thigh strap. In particular, the upper leg comprises a seat unit configured for providing at least one sitting surface for the person, in particular in case the person is sitting or partly sitting on the wearable sitting posture assisting device, preferably for the thigh and/or at least a lower portion of a buttock of the person, wherein "buttock" is preferably to mean one cheek of the buttocks. Preferably, the seat unit comprises at least one sitting element which features the sitting surface. Advantageously, the seat unit is in contact with the thigh of the person in case the person is sitting or partly sitting on the wearable sitting posture assisting device. Preferably, the seat unit is arranged on a rear side of the thigh of the person in case the person is standing or walking with the wearable sitting posture assisting device.

Advantageously, the upper leg comprises at least one upper leg support. Preferably, the seat unit is connected to the upper leg support. Advantageously, the thigh connection unit and/or the thigh strap is connected to the upper leg support. In particular, the upper leg support is implemented as a frame element. Preferably, the upper leg support is implemented as an elongate element. Advantageously, the upper leg features at least one upper leg longitudinal axis which is oriented at least substantially parallel to a longitudinal axis of the thigh of the person. Preferably, a main extension direction of the upper leg support is oriented at least substantially parallel, or parallel, to the upper leg longitudinal axis. In particular, the upper leg support is at least partly, preferably at least to a large extent, advantageously completely made of plastic. It is also conceivable that the upper leg support is at least partly, preferably at least to a large extent, advantageously completely made of metal, in particular made of a light metal or a light alloy, for instance aluminum and/or titanium and/or beryllium and/or scandium or other suitable metals. It is further conceivable that the upper leg support is at least partly, preferably at least to a large extent, advantageously completely made of a composite material, in particular a fiber reinforced composite material and/or a fiber reinforced plastic and/or a carbon fiber reinforced material and/or a carbon fiber reinforced polymer and/or a fiber reinforced thermoplastic. The term "at least to a large extent" is in particular to mean to an extent of at least 55 %, preferably to an extent of at least 65 %, further preferably to an extent of at least 75 %, advantageously to an extent of at least 85 % and further advantageously to an extent of at least 95 %. In this context "at least substantially parallel" is in particular to be understood as an orientation of a direction with respect to a reference direction, in particular in a plane, wherein the direction has a deviation from the reference direction in particular of less than 15º, advantageously of less than 10º and particularly advantageously of less than 2º. A "main extension direction" of an object is, in particular, to be understood, in this context, as a direction extending in parallel to a largest side of an imaginary rectangular cuboid which only just entirely encloses the object.

Preferably, the lower leg is arranged on a rear side of the shank of the person in at least one wearing state. In particular, the lower leg comprises at least one lower leg support. Advantageously, the lower leg support is implemented as a frame element. Preferably, the lower leg support is implemented as an elongate element. In particular, the lower leg features at least one lower leg longitudinal axis which is oriented at least substantially parallel to a longitudinal axis of the shank of the person. Preferably, a main extension direction of the lower leg support is oriented at least substantially parallel, or parallel, to the lower leg longitudinal axis. Advantageously, the lower leg longitudinal axis is oriented at least substantially parallel, or parallel, to the upper leg longitudinal axis.

Preferably, the upper leg and the lower leg together define a sitting angle. Advantageously, the sitting angle is an angle included between the upper leg longitudinal axis and the lower leg longitudinal axis, in particular on a rear side of the upper leg and the lower leg. In particular, in the sitting posture the sitting angle is no greater than 130°, preferably no greater than 120° and advantageously no greater than 110° and/or no smaller than 60°, preferably no smaller than 70° and advantageously no smaller than 80°. In particular, the sitting angle is approximately 90° in the sitting posture. In particular, in the partly sitting posture the sitting angle is no greater than 170°, preferably no greater than 160° and advantageously no greater than 150° and/or no smaller than 100°, preferably no smaller than 110° and advantageously no smaller than 120°. In particular, the sitting angle is approximately 130° in the partly sitting posture. Preferably, the sitting angle equals the angle between the thigh and the shank of the person. In particular, the sitting angle is approximately 180° in a standing posture.

In particular, the knee joint pivotably connects the lower leg to the upper leg, preferably pivotably about a knee joint axis. Advantageously, the knee joint axis is oriented at least substantially perpendicularly, or perpendicularly, to the upper leg longitudinal axis and/or to the lower leg longitudinal axis. Preferably, the knee joint axis is oriented at least substantially perpendicularly, or perpendicularly, to the sitting direction. Advantageously, the upper leg support and the lower leg support together implement at least a portion of the knee joint, or the knee joint. Preferably, a value of the sitting angle corresponds to a value of a knee joint position of the knee joint. In this context "at least substantially perpendicularly" is in particular to mean an orientation of a direction with respect to a reference direction, in particular in a plane, wherein the direction and the reference direction include an angle which deviates from an angle of 90° by no more than 15º, advantageously by no more than 10º and particularly advantageously by no more than 2º.

Preferably, the wearable sitting posture assisting device comprises at least one locking unit, which is configured for locking the upper leg with respect to the lower leg and/or the knee joint in a certain sitting angle and/or is configured for defining a smallest sitting angle. Advantageously, the locking unit is configured for locking the knee joint in different sitting angles, and/or for defining different smallest sitting angles, which sitting angles can be preferably chosen by the person. It is conceivable that the locking unit is configured for allowing to increase the sitting angle in a locked state. In particular, the person is enabled to stand up when the locking unit is in the locked state. Preferably, the locking unit is configured for enabling the person to sit down again at the defined smallest sitting angle after standing up with the locking unit still defining the same smallest sitting angle. Advantageously, the locking unit comprises at least one blocking element which is configured for blocking and/or unblocking the knee joint and/or for fixating the sitting angle and/or for defining a smallest sitting angle. Preferably, the blocking element is implemented as a spring, in particular as a gas spring. Advantageously, the blocking element is connected to the upper leg, in particular to the upper leg support, and to the lower leg, in particular to the lower leg support. Preferably, the blocking element is configured for damping a movement of the upper leg with respect to the lower leg during sitting down and/or during standing up. Advantageously, the locking unit features at least one actuation element, which is configured for actuating the blocking element. Preferably, the actuation element is configured for enabling the person to block or unblock the blocking element. Advantageously, the actuation element is a mechanical actuation element. It is also conceivable that the actuation element is an electronic actuation element. It is further conceivable that the locking unit comprises at least one control unit which is configured for detecting a sitting-down condition when the person sits down and/or detecting a standing up condition when the person stands up and/or triggering the actuation element according to a requirement for blocking the blocking element.

In particular, the ground contact unit comprises at least one ground contact element. Preferably, the ground contact element features at least one ground contact surface, which is advantageously configured for contacting a ground when the person is sitting or partly sitting on the wearable sitting posture assisting device. Advantageously, the ground contact surface is bent and/or curved, in particular convexly bent and/or convexly curved. However, it is also conceivable that at least a portion of or the entire ground contact surface is at least substantially flat or flat. In particular, the ground contact element is at least partly, preferably at least to a large extent, advantageously completely made of rubber. Preferably, a weight force of the person is transmitted from the seat unit to the upper leg support and/or from the upper leg support to the knee joint and/or from the knee joint to the lower leg support and/or from the lower leg support to the ground contact element and/or from the ground contact element to the ground. In particular, the weight of the person is additionally transmitted to the ground via the foot and/or shoe of the person. Preferably, the ground contact element is arranged on a rear side of the shoe and/or the foot of the person. When the person is sitting or partly sitting on the wearable sitting posture assisting device, the foot and/or the shoe of the person is in contact with the ground in addition to the ground contact element. Preferably, the ground contact element is arranged contactlessly with respect to the ground when the person is walking or standing while wearing the sitting posture assisting device.

Advantageously, the body connection unit is configured for at least partly connecting at least a portion of the wearable sitting posture assisting device, in particular one leg unit, to the person in the wearing state. Preferably, a position of the body part connector with respect to the thigh and/or shank of the person it is connected to is at least substantially fixed, or fixed, in the wearing state. In particular, the body part connector is configured for connecting to the thigh and/or the shank of the person such that its position with respect to the thigh and/or shank of the person it is connected to changes by no more than 10 cm, preferably by no more than 5 cm and further preferably by no more than 3 cm while the person is wearing the wearable sitting posture assisting device. Preferably, the body part connector comprises at least one body connection element for connecting to the thigh and/or shank of the person, in particular to a piece of clothing. The body connection element may for instance comprise at least one strap element, at least one bracket element, at least one clamp element, at least one fastening element and/or the like. It is conceivable that the body part connector is implemented integrally. It is also conceivable that the body part connector is implemented modularly, in particular with the body connection element being implemented as an exchangeable modular portion of the body part connector, which advantageously allows an adjustment to at least one body measurement of the person wearing the wearable sitting posture assisting device. Preferably, the body part connector is configured for connecting either to a shank or to a thigh of the person. It is also conceivable that the body part connector is configured for connecting to a thigh and a shank of the person, in particular to a knee and/or a knee region of the person.

In this context, a first object being "movably mounted" to a second object is in particular to mean that the first object is movable in one, two or three dimensions with respect to the second object within a defined, in particular macroscopic, region, wherein a position of the first object is in particular restricted to be within the region due to at least one mounting of the first object to the second object, and wherein in particular the region is larger than a region defined only by tolerances of a fixed and/or rigid mounting. In particular, the first object is mounted and/or connected and/or in particular permanently connected to the second object in such a way that it is movable with respect to the second object. Advantageously, the body part connector is movably supported by the support element. Preferably, the body part connector is movable in at least one direction and/or at least partly rotatable and/or pivotable about at least one rotation axis and/or pivot axis in a mounted state. Further preferably, the body part connector is in the mounted state movable over a distance of at least 5 cm, preferably of at least 10 cm, further preferably of at least 15 cm and advantageously of at least 20 cm. Advantageously, a position of the body part connector with respect to the support element is at least substantially fixed or fixed in at least one direction, in which the body part connector is in particular movable over no more than 10 mm, preferably over no more than 5 mm and further preferably over no more than 3 mm. The term "mounted" in the context of this disclosure is, in particular, to mean preferably permanently fixed and/or fixated to and/or attached to and/or fastened to.

In particular, a position of the support element with respect to the leg unit, in particular with respect to the upper leg and/or to the lower leg, is at least substantially fixed or fixed. Preferably, the support element is rigid. It is also conceivable that the support element is at least partly macroscopically deformable and/or reversibly deformable. It is conceivable that the support element is at least partly, preferably at least to a large extent, made of metal, in particular aluminum and/or titanium and/or scandium and/or steel, and/or stainless steel or the like, and/or plastic and/or a composite material, for instance a carbon fiber material. In this context, a "macroscopically deformable object" is, in particular, to mean an object having at least one extension in at least one direction, which extension can be, in particular temporarily and/or without damage, altered by at least 1 %, preferably by at least 5 %, further preferably by at least 20 % and advantageously by at least 50 % when a force is applied to the object that is no greater than 1000 N mm⁻², preferably no greater than 100 N mm⁻² and advantageously no greater than 10 N mm⁻².

In a further embodiment of the invention it is proposed that the body part connector is movable in a direction that is at least substantially parallel to a main extension direction of the support element. Preferably, the body part connector is mounted in such a way that it is movable only in a direction that is at least substantially parallel to the main extension direction of the support element. Advantageously, the main extension direction of the support element is oriented at least substantially parallel to the lower leg longitudinal axis and/or at least substantially parallel to the upper leg longitudinal axis. In particular, the body connection unit is configured for allowing a movement of the body part connector with respect to the support element, which is in particular at least substantially parallel to the main extension direction of the support element when the person is sitting down and/or standing up and/or changing a sitting posture and/or a sitting angle. As a result, a movement of a body part connector with respect to a leg unit is possible when changing a sitting or standing posture. Furthermore, a high degree of wearing comfort can be achieved, in particular when moving a body part while wearing a wearable sitting posture assisting device.

A wearable sitting posture assisting device providing a stable sitting support can be provided and/or an intuitive handling can be achieved, in particular, if the body part connector is slidably mounted to the support element and/or slidably supported by the support element. In particular, the body part connector is slidable in the direction that is at least substantially parallel to the main extension direction of the support element. It is conceivable that the body part connector is freely slidable with respect to the support element. Alternatively, the body connection unit may comprise at least one slowing unit, which in particular comprises at least one slowing element, preferably a friction element, which is configured to impede a sliding of the body part connector along the support element. It is conceivable that the slowing unit is configured to provide an adjustable degree of slowing for the sliding movement, which degree is in particular adjustable by the person. Additionally or alternatively it is conceivable that the support element comprises at least one sliding surface, which is in contact with the body part connector at least in a sliding state of the body part connector. The sliding surface may feature a low friction coefficient or a high friction coefficient, in particular depending on whether or not an unimpeded or mostly unimpeded sliding of the body part connector is desired or slowing of a movement is desired. It is conceivable that the body connection unit comprises at least one exchangeable sliding layer element featuring the sliding surface, which in particular results in an adjustability of friction properties.

It is further proposed that the support element comprises at least one rail element. Preferably, the support element is implemented as a bracket and/or is bar-handle-shaped. Advantageously, the support element is implemented as the rail element. It is conceivable that the rail element is implemented integrally. Preferably, the rail element comprises at least one first connection element for connecting to at least one first connection point of the leg unit and comprises in particular at least one second connection element for connecting to at least one second connection point of the leg unit. Advantageously, the first connection element and the second connection element are arranged on opposite sides, in particular ends, of the support element, in particular along its main extension direction. As a result, a high degree of mechanical reliability and/or wear-resistance can be achieved.

Controlled and/or favorable sliding properties can be achieved, in particular, if the body connection unit comprises at least one sliding element, which connects the body part connector to the support element. Advantageously, the sliding element is slidable with respect to the support element. In particular, a sliding movement of the body part connector corresponds to a sliding of the sliding element with respect to the support element and/or along the support element, in particular in its main extension direction. It is conceivable that the body part connector comprises the sliding element. It is further conceivable that the body part connector is implemented integrally with the sliding element. It is also conceivable that the body part connector is connected to the sliding element, at least in the wearing state. Preferably, the body connection element is in the wearing state connected to the sliding element. Furthermore, it is conceivable that the sliding element comprises the slowing unit. Preferably, the sliding element defines at least one link region for linking to the support element. Advantageously, the sliding element movably mounts the body part connector to the support element, in particular via the link region. In particular, the support element protrudes in the wearing state through the link region. Advantageously, a cross section of the link region corresponds to a cross section of the support element, in particular to a cross section of the rail element.

It is further proposed that the body connection unit comprises at least one reversibly deformable compensation element configured for generating at least one restoring force for the body part connector, which in particular acts upon the body connection element in at least one deflected state of the body part connector, in particular in at least one deflected state of the body connection element. In particular, the person exerts in the deflected state at least one deflecting force onto the body connection unit, for instance when moving a foot away from the ground contact element when sitting or partly sitting on the wearable sitting posture assisting device. Preferably, the body connection element is deflected with respect to a basic position of the body connection element, in particular regarding a distance between the body connection element and the support element parallel to the sitting direction, in a direction that is perpendicular to the main extension direction of the support element and/or in a direction that is perpendicular to a surface normal of a plane defined by the main extension direction of the support element and the sitting direction. Advantageously, the compensation element defines a maximum range for a possible movement of the body connection element in a direction that is perpendicular to the main extension direction of the support element and in particular parallel to the sitting direction. Preferably, the body connection element is movable farthest in a direction that is at least substantially parallel to the main extension direction of the support element, and/or second farthest in a direction that is at least substantially parallel to the sitting direction, and/or the least far in a direction that is at least substantially perpendicular to the main extension direction of the support element and to the sitting direction. Advantageously, the compensation element is configured for restoring the basic position of the body connection element when the person is standing up and/or walking with the wearable sitting posture assisting device. As a result, a stable sitting position may be maintained while moving a foot. Advantageously, a high degree of sitting comfort can be achieved, in particular since feet can be easily moved while sitting on a wearable sitting posture assisting device in order to avoid numbness and/or a tingling sensation or the like in a foot and/or a leg.

A high degree of comfort when putting on and/or taking off the wearable sitting posture assisting device can be achieved, in particular, if the body part connector is connectable to and/or disconnectable from the support element, in particular without tools. Preferably, the body part connector is entirely disconnectable from the support element. In particular in this case, the person is advantageously enabled to put on and/or take off the body part connector in a disconnected state, whereby freedom of movement can be achieved. Preferably, the body part connector comprises the sliding element, which is connectable to and/or disconnectable from the support element. It is also conceivable that the body part connector and/or the body connection element is connectable to and/or disconnectable from the sliding element, and in particular that the sliding element is permanently movably mounted to the support element.

In particular, a connection between the body part connector and the support element in the connected state is configured for withstanding a force, in particular a pulling force exerted onto the body part connector, in particular in a direction perpendicular to a main extension plane of the connection, of at least 50 N, preferably of at least 100 N, further preferably of the least 200 N, advantageously of at least 300 N and further advantageously of at least 400 N. It is conceivable that a maximum connection force of the connection of the body part connector and the support element is adjustable, for instance allowing to adjust a connection force to a weight and/or a strength of the person and/or to a area of application of the wearable sitting posture assisting device. Advantageously, the body part connector is entirely disconnected from the support element in a disconnected state of the body part connector. It is conceivable that the body connection unit, in particular the support element and/or the body part connector, comprises at least one actuation element for locking and/or unlocking the connection between the body part connector and the support element. In particular, connecting the body part connector to the support element may encompass bringing the body part connector in contact with the support element and/or fastening the body part connector to the support element and/or locking the connection. It is also conceivable that the body part connector and/or the support element implements or together implement at least one self-locking and/or self-unlocking connection. In particular, the connection between the body part connector and the support element may be configured for self-unlocking and/or for self-releasing in case a threshold force exerted onto the body part connector is exceeded and/or in an emergency case. It is further conceivable that the threshold force is adjustable. Furthermore, it is conceivable that the body connection unit comprises at least one sensor unit for detecting at least one emergency condition. In particular in this case the body connection unit may comprise at least one automatic actuation unit for releasing the connecting of the body part connector to the support element in an, in particular detected, emergency condition. As a result, a high degree of safety can be achieved and/or injuries can be avoided. A "main extension plane" of an object is, in particular, to be understood as a plane extending parallel to a largest side of an imaginary rectangular cuboid which only just entirely encloses the object and preferably extends through a geometric center of the object.

In particular, the body part connector is connected to the body part of the person in the body part connector wearing state. In the body part connector wearing state the body part connector may be connected or disconnected from the support element. Preferably, in at least one normal wearing condition of the wearable sitting posture assisting device a force and/or a movement of the body part is at least partly transmitted from the body part connector to the support element and/or from the support element to the leg unit.

Fast and/or easy putting on and/or taking off a wearable sitting posture assisting device can be achieved, in particular, if the body part connector, preferably the body connection element, comprises at least one quick fastener. For instance, the quick fastener may be implemented as a magnetic quick fastener, a click-in quick fastener, a hook-and-loop quick fastener, an electromagnetic quick fastener, a quick fastener featuring at least one positive-locking fastening element and/or a quick fastener featuring at least one force-fitting fastening element, or the like. It is further conceivable that the body part connector comprises at least one actuation element for the quick fastener, in particular a release actuation element.

In a further embodiment of the invention it is proposed that the body part connector comprises at least one ventilation section, which is at least partially air-permeable. In particular, the ventilation section may comprise at least one opening through which the thigh or leg of the person is in contact with air of an environment. It is also conceivable that the ventilation section comprises at least one ventilation material, for instance a mesh-like material and/or a porous material and/or a microporous material and/or a breathable material. As a result, a high degree of wearing comfort can be achieved. Furthermore, an encumbrance of a person wearing a wearable sitting posture assisting device due to sweating can be reduced.

In another embodiment of the invention it is proposed that the body part connector comprises at least one padding element. Preferably, the padding element comprises a surface facing away from the support element. It is conceivable that the padding element is part of the ventilation section and/or implements the ventilation section. As a result, a high degree of comfort, in particular when sitting or partly sitting on and/or when walking with the wearable sitting posture assisting device, is achievable.

In yet another embodiment of the invention it is proposed that the body part connector is implemented as a shank connector, in particular configured for connection to the shank of the person. Preferably, the body connection element is implementing as a shank strap and/or as a shank bracket and/or as another shank connection element. In particular in this case, it is conceivable that the wearable sitting posture assisting device is free of a foot connector. Preferably, in the wearing state the leg unit is connected to the respective leg of the person only via the thigh connection unit and the body part connector. Further preferably, a position of the thigh connection unit, in particular of a thigh connector of the thigh connection unit, is fixed with respect to the leg unit. As a result, a high stability in combination with a high degree of comfort can be achieved when supporting a sitting or partly sitting posture.

It is also conceivable that the body part connector is implemented as a thigh connector and/or that the body connection unit implements at least a portion of the thigh connection unit. In particular in this case, it is conceivable that the support element is mounted to the upper leg, in particular to the upper leg support. The support element may also be at least partly implemented integrally with the upper leg, in particular with the upper leg support. In particular in this case, it is conceivable that the wearable sitting posture assisting device, in particular the leg unit, comprises the foot connector. Additionally or alternatively, in particular in this case, it is conceivable that the wearable sitting posture assisting device, in particular the leg unit, comprises a shank connector, wherein preferably a position of the shank connector is fixed with respect to the leg unit. Furthermore, it is conceivable that the body part connector is at least a portion of the foot unit and/or that the body connection unit implements at least a portion of the foot unit. As a result, moving a thigh is possible while sitting or partly sitting on a wearable sitting posture assisting device.

In particular in case the body part connector is implemented as a thigh connector, the leg unit may comprise the foot unit and the foot unit may be configured for connecting to a shoe and/or to a foot of the person, and/or the foot unit is connected to a shoe and/or to a foot of the person. In particular, the foot unit may comprise at least one shoe connector for connecting to the foot and/or to the shoe of the person. It is conceivable that the foot connector features at least one shoe strap. In particular, the foot connector may feature at least one upper strap, which advantageously runs across an instep of the foot or of the shoe the foot unit is connected to. Preferably, the foot connector may feature at least one lower strap, which advantageously runs across a sole of the foot or of the shoe the foot unit connected to. In particular, the foot connector may be configured for being worn on a shoe and/or on a foot. Advantageously, the foot unit may comprise at least one foot unit support. Preferably, the shoe connector may be connected to the foot unit support. Advantageously, the foot unit support may comprise at least one bracket and/or the foot unit support may be implemented as a bracket. Preferably, the shoe strap, in particular the upper strap and/or the lower strap, may be connected to the bracket. It is conceivable that the foot unit support is at least partly implemented integrally, or implemented integrally, with the lower leg, in particular with the lower leg support.

A high degree of wearing comfort and/or handling comfort of a leg unit can be achieved, in particular, if the body connection unit is mounted to the leg unit. Preferably, the support element is mounted to the leg unit. It is conceivable that the leg unit implements the support element and/or is at least partly implemented integrally with the support element.

It is further proposed that the body connection unit, in particular the support element, is mounted to the lower leg, in particular to the lower leg support. It is conceivable that the support element is at least partly implemented integrally with the lower leg, in particular with the lower leg support. Preferably in this case it is conceivable that the body part connector is implemented as a shank connector or as a foot connector. As a result, a safe and comfortable connection between a leg unit and a leg of a person wearing the leg unit can be achieved, in particular while enabling the person to move his foot while sitting or partly sitting.

The invention further encompasses a method of putting on or taking off the wearable sitting posture assisting device, wherein the body part connector is connected to or disconnected from the thigh and/or the shank of the person in a disconnected state, in particular in a state in which the body part connector is disconnected from the support element. Preferably, the body part connector is connected to and/or disconnected from the support element and/or the sliding element in at least one body part connector wearing state, in which, in particular, the person is wearing the body part connector.

Herein, the wearable sitting posture assisting device and the method according to the invention are not to be limited to the application and implementation described above. In particular, for the purpose of fulfilling a functionality herein described, the wearable sitting posture assisting device and the method according to the invention may comprise a number of respective elements, structural components, units and/or steps that differ from the number mentioned herein. Furthermore, regarding the value ranges mentioned in this disclosure, values within the limits mentioned are to be understood to be also disclosed and to be used as applicable.

### Drawing

Further advantages may become apparent from the following description of the drawing. In the drawing exemplary embodiments of the invention are shown. The drawing, the description and the claims contain a plurality of features in combination. The person having ordinary skill in the art will purposefully also consider the features separately and will find further expedient combinations.

If there is more than one specimen of a certain object, in some instances only one of these may be given a reference numeral in the figures and in the description. The description of this specimen may be correspondingly transferred to the other specimens of the object.

It is shown in:
- Fig. 1: a person wearing a wearable sitting posture assisting device, in a schematic lateral view,
- Fig. 2: the person wearing the wearable sitting posture assisting device, in a schematic front view,
- Fig. 3: the person wearing the wearable sitting posture assisting device in a schematic rear view,
- Fig. 4: a portion of the wearable sitting posture assisting device comprising a body connection unit in a first wearing state, in a schematic lateral view,
- Fig. 5: a support element of the body connection unit, in a perspective view,
- Fig. 6: the support element, in a schematic sectional view,
- Fig. 7: a body part connector of the body connection unit, in a perspective view,
- Fig. 8: the portion of the wearable sitting posture assisting device in a disconnected state of the body connection unit, in a schematic lateral view,
- Fig. 9: the portion of the wearable sitting posture assisting device in a second wearing state, in a schematic lateral view,
- Fig. 10: a first alternative body connection unit, in a perspective view,
- Fig. 11: a second alternative body connection unit, in a perspective view,
- Fig. 12: a body part connector of the second alternative body connection unit, in a perspective front view,
- Fig. 13: the body part connector of the second alternative body connection unit, in a perspective rear view and
- Fig. 14: a third alternative body connection unit, in a perspective view.

### Description of the exemplary embodiments

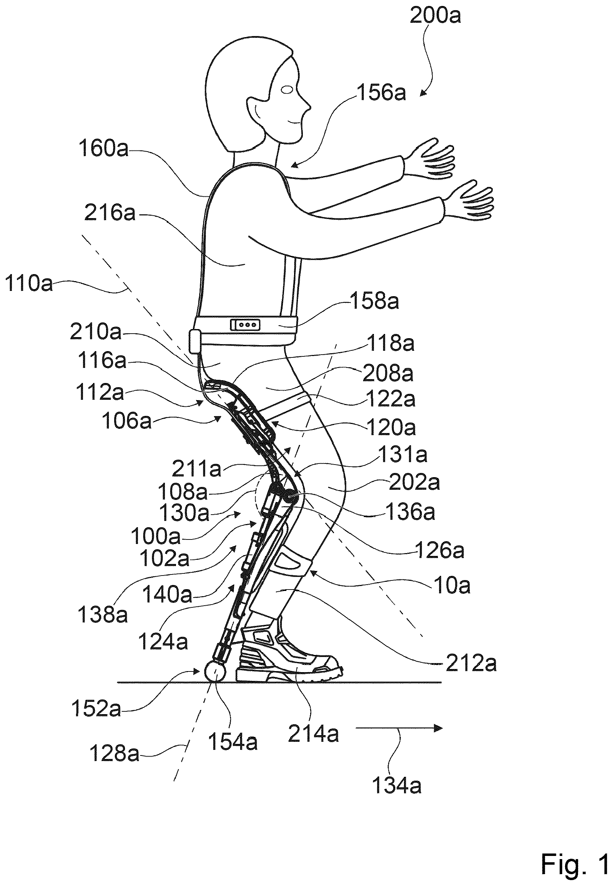

Fig. 1 shows a person 200a wearing a wearable sitting posture assisting device 100a. The wearable sitting posture assisting device 100a is configured for receiving a weight force of the person 200a in a sitting posture or in a partly sitting posture. In fig. 1 the person 200a is shown in a partly sitting posture. In the partly sitting posture a knee 202a of the person is partly bent. In a sitting posture the knee 202a is bent more strongly than in the partly sitting posture. The wearable sitting posture assisting device 100a is configured for allowing the person 200a to sit down on it in different sitting postures and in different partly sitting postures. Furthermore, the wearable sitting posture assisting device 100a is configured for allowing the person 200a to walk while wearing the wearable sitting posture assisting device 100a. In addition, the wearable sitting posture assisting device 100a is configured for allowing the person 200a to stand and/or stand up and/or sit down and/or walk while wearing the wearable sitting posture assisting device 100a.

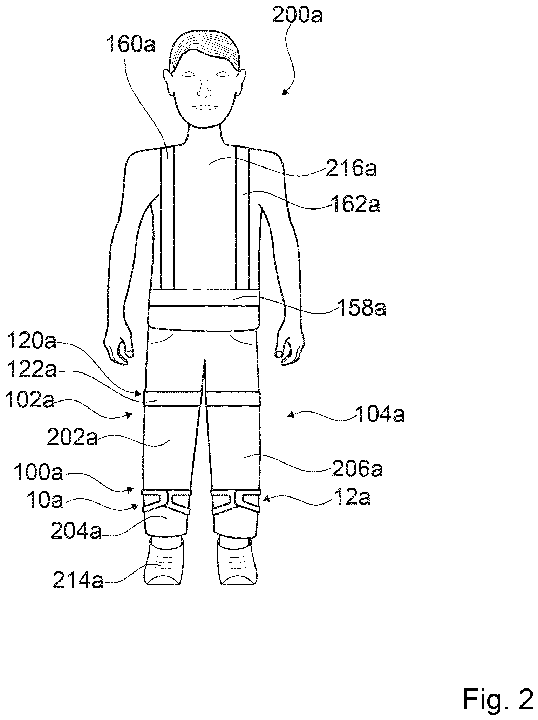

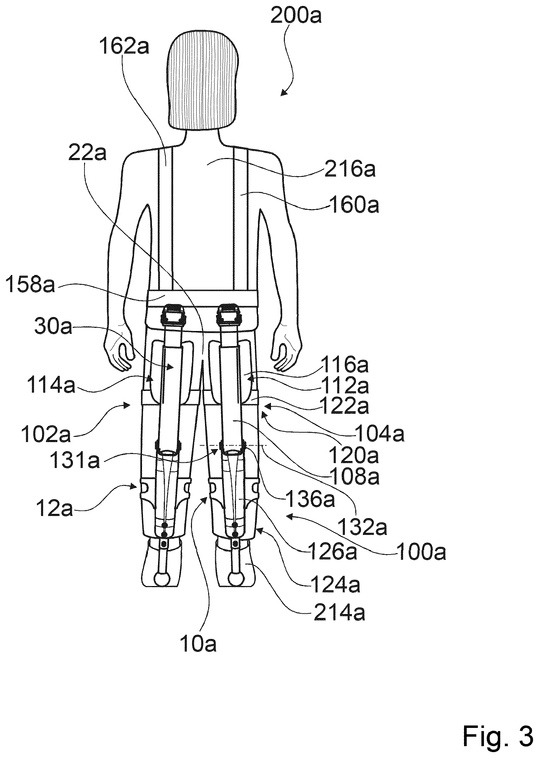

Fig. 2 shows the person 200a wearing the wearable sitting posture assisting device 100a, in a schematic front view. Fig. 3 shows the person 200a wearing the wearable sitting posture assisting device 100a, in a schematic rear view. In figs 1 to 3 the wearable sitting posture assisting device 100a is shown in a normal wearing condition. The normal wearing condition encompasses a condition in which the person 200a is sitting or partly sitting on the wearable sitting posture assisting device 100a, a condition in which the person 200a is standing up, a condition in which the person 200a is sitting down, a condition in which the person 200a is standing, and a condition in which the person 200a is walking, in each case while wearing the wearable sitting posture assisting device 100a. In the case shown the person 200a is wearing the wearable sitting posture assisting device 100a in a factory building, in particular while working at an assembly line. In a similar fashion it is conceivable that the person 200a wears the wearable sitting posture assisting device 100a in an office building, in a factory building, in a service building, outdoors, at work, at home, while working, during breaks, etc. Advantageously, the person 200a wears the wearable sitting posture assisting device 100a during an activity which requires the person 200a to sit down and/or to partly sit down and/or to stand up and/or to stand and/or to walk repeatedly. The person 200a can then sit down on the wearable sitting posture assisting device 100a when required, stand up while wearing the wearable sitting posture assisting device 100a when required, and walk while wearing the wearable sitting posture assisting device 100a when required.

The wearable sitting posture assisting device 100a comprises a leg unit 102a. Furthermore, the wearable sitting posture assisting device 100a comprises an additional leg unit 104a. The additional leg unit 104a is implemented identically to the leg unit 102a. Therefore, in the following only the leg unit 102a is described in detail. The description of the leg unit 102a is to be understood as transferable to the additional leg unit 104a. It is also conceivable that an additional leg unit is implemented mirror-symmetrically to the leg unit. In particular, it is conceivable that a leg unit and an additional leg unit are implemented as a right leg unit and a left leg unit, respectively, or vice versa.

In the case shown, the person 200a wears the leg unit 102a on a right leg 204a. The leg unit 102a is arranged on a rear side 211 a of the leg 204a of the person 200a. Furthermore, the person 200a wears the additional leg unit 104a on a left leg 206a. It is also conceivable that a person wears a leg unit on a left leg and an additional leg unit on a right leg. Furthermore, it is conceivable that a person only wears one leg unit. In addition, it is conceivable that a wearable sitting posture assisting device comprises only one leg unit. It is also conceivable that a leg unit is arranged on a lateral side of a leg and/or on a front side of a leg and/or between two legs of a person.

The person 200a sits or partly sits on the wearable sitting posture assisting device 100a in a sitting direction 134a. The person 200a faces and/or looks in the sitting direction 134a when facing forward. The sitting direction 134a is oriented parallel to a ground on which the person 200a is sitting or walking or standing.

The leg unit 102a comprises an upper leg 106a. The upper leg 106a comprises an upper leg support 108a. The upper leg 106a has an upper leg longitudinal axis 110a. The upper leg longitudinal axis 110a is oriented parallel to a plane defined by the sitting direction 134a and a surface normal of a ground the wearable sitting posture assisting device 100a is standing on in the normal wearing condition. The upper leg support 108a has a main extension direction which is oriented parallel to the upper leg longitudinal axis 110a. The upper leg longitudinal axis 110a is oriented parallel to a main extension direction of a thigh 208a of the leg 204a of the person, in particular when the person 200a is sitting, and/or partly sitting and/or walking and/or standing up and/or standing while wearing the wearable sitting posture assisting device 100a.

The upper leg 106a comprises a seat unit 112a. The seat unit 112a is connected to the upper leg support 108a. In the partly sitting posture and/or in the sitting posture the person 200a sits on the seat unit 112a. In the case shown the person 200a sits on the seat unit 112a and on a seat unit 114a of the additional leg unit 104a in the partly sitting posture. The seat unit 112a comprises a sitting element 116a. The sitting element 116a contacts the thigh 208a of the person 200a. Furthermore, in the sitting posture and/or in the partly sitting posture the sitting element 116a contacts a buttock 210a of the person 200a. The seat unit 112a comprises a sitting surface 118a. The sitting element 116a comprises the sitting surface 118a. The sitting surface 118a is configured for allowing the person 200a to sit down on it with the thigh 208a and/or with the buttock 210a. A shape of the sitting surface 118a is at least partly adjusted to the thigh 208a and/or to the buttock 210a of the person 200a. The sitting surface 118a is curved. The sitting surface 118a is concavely curved and/or bent.

It is also conceivable that a wearable sitting posture assisting device comprises only one seat unit, in particular a common seat unit of two leg units. In particular, in this case it is conceivable that the seat unit is saddle-shaped and/or implemented in the manner of a saddle, in particular arranged between the legs of a person.

The upper leg 106a comprises a thigh connection unit 120a for connecting to the thigh 208a of the person 200a. The thigh connection unit 120a is connected to the upper leg support 108a. The thigh connection unit 120a is configured for connecting the upper leg 106a to the thigh 208a of the person 200a. The thigh connection unit 120a comprises a thigh strap 122a. The thigh strap 122a is fixed to the thigh 208a of the person 200a.

The wearable sitting posture assisting device 100a comprises a lower leg 124a. The lower leg 124a comprises a lower leg support 126a. The lower leg 124a has a lower leg longitudinal axis 128a. The lower leg longitudinal axis 128a is oriented parallel to a plane defined by the sitting direction 134a and a surface normal of a ground the wearable sitting posture assisting device 100a is standing on in the normal wearing condition. The lower leg longitudinal axis 128a and the upper leg longitudinal axis 110a are arranged in a common plane. The lower leg support 126a has a main extension direction which is oriented parallel to the lower leg longitudinal axis 128a. The lower leg longitudinal axis 128a is oriented parallel to a main extension direction of a shank 212a of the leg 204a of the person 200a, in particular when the person 200a is sitting, and/or partly sitting and/or walking and/or standing while wearing the wearable sitting posture assisting device 100a.

The upper leg 106a and the lower leg 124a define a sitting angle 130a. The sitting angle 130a is an angle included by the upper leg longitudinal axis 110a and the lower leg longitudinal axis 128a. The sitting angle 130a is similar or identical to an angle between the thigh 208a and the shank 212a of the person 200a. The sitting angle 130a having a value between 60° and 130°, in particular a value of approximately 90°, corresponds to different sitting postures or to at least one sitting posture. The sitting angle 130a having a value between 130° and 170° corresponds to different partly sitting postures. In case the person 200a is standing while wearing the wearable sitting posture assisting device 100a the sitting angle 130a has a value between 160° and 180°, in particular a value of approximately 180°. In case the person 200a is walking while wearing the wearable sitting posture assisting device 100a the sitting angle 130a may significantly differ from 180°, in particular in case the person 200a bends his knee 202a. In the sitting posture and/or in the partly sitting posture and/or when standing the sitting angle 130a and an analogously defined additional sitting angle of the additional leg unit 104a are advantageously identical. However, it is also conceivable that the person 200a may sit on the wearable sitting posture assisting device 100a in a sitting posture or a partly sitting posture with the sitting angle 130a and the additional sitting angle differing, in particular by up to 5°, by up to 10°, by up to 15°, by up to 20°, by up to 30°, by up to 40°, or more. When the person 200a is walking while wearing the wearable sitting posture assisting device 100a, the sitting angle 130a and the additional sitting angle may significantly differ, for instance in case the person 200a bends one of his knees 202a more than his other knee 202a.

The leg unit 102a comprises a knee joint 131 a, which pivotably connects the upper leg 106a to the lower leg 124a. The knee joint 131 a connects the upper leg 106a to the lower leg 124a pivotably about a knee joint axis 132a. The knee joint axis 132a is oriented perpendicularly with respect to the upper leg longitudinal axis 110a. The knee joint axis 132a is oriented perpendicularly to the lower leg longitudinal axis 128a. The knee joint axis 132a is oriented perpendicularly to the sitting direction 134a. The knee joint 131 a is partly implemented integrally with the upper leg support 108a. The knee joint 131 a is partly implemented integrally with the lower leg support 126a. The knee joint 131 a comprises at least one bearing 136a, which connects the upper leg support 108a to the lower leg support 126a.

The leg unit 102a comprises a locking unit 138a which is configured for locking the knee joint 131 a. The locking unit 138a is configured for limiting the sitting angle 130a to a minimum value. The locking unit 138a is configured for allowing the person 200a to choose the minimum value of the sitting angle 130a. In case the locking unit 138a is in a locked state, the person 200a can sit down on the wearable sitting posture assisting device 100a with the sitting angle 130a at the minimum value. The locking unit 138a is configured for being actuated by the person 200a. The locking unit 138a comprises a blocking element 140a. The blocking element 140a is a spring, in particular a gas spring. The blocking element 140a is configured for being lockable at different lengths. The blocking element 140a is connected to the upper leg support 108a. The blocking element 140a is connected to the lower leg support 126a. The blocking element 140a is configured for damping a movement of the upper leg 106a with respect to the lower leg 124a, in particular when the person 200a is sitting down.

The leg unit 102a comprises a ground contact unit 152a. The ground contact unit 152a is connected to the lower leg support 126a. The ground contact unit 152a comprises a ground contact element 154a. When the person 200a is sitting or partly sitting on the wearable sitting posture assisting device 100a, the ground contact unit 152a, in particular the ground contact element 154a, is in contact with the ground. The ground contact unit 152a, in particular the ground contact element 154a, is configured for transmitting a portion of the weight force of the person 200a to the ground. The ground contact element 154a is made of rubber. However, other shapes and/or materials are conceivable for a ground contact element as mentioned above.

In case the person 200a is sitting or partly sitting on the wearable sitting posture assisting device 100a, the weight force of the person 200a is at least partly, in particular directly or indirectly, transmitted from the seat unit 112a to the upper leg support 108a; from the upper leg support 108a to the knee joint 131 a; from the knee joint 131 a to the lower leg support 126a; from the lower leg support 126a to the ground contact element 154a; and from the ground contact element 154a to the ground.

In particular, the weight force of the person 200a is additionally transmitted to the ground via a foot and/or shoe 214a of the person 200a. Preferably, the ground contact element 154a is arranged on a rear of the shoe 214a of the person 200a.

When the person 200a sits or partly sits on the wearable sitting posture assisting device 100a, the foot and/or shoe 214a of the person 200a is in contact with the ground in addition to the ground contact element 154a. Preferably, the ground contact element 154a is arranged contactlessly with respect to the ground when the person 200a is walking and/or standing while wearing the wearable sitting posture assisting device 100a.

The wearable sitting posture assisting device 100a comprises an upper body wearing unit 156a. The person 200a wears the upper body wearing unit 156a on his upper body 216a, which upper body 216a may include hips and/or a waist of the person 200a. The upper body wearing unit 156a comprises a belt 158a. Furthermore, the upper body wearing unit 156a comprises suspenders 160a, 162a. The leg unit 102a is connected to the upper body wearing unit 156a. The additional leg unit 104a is connected to the upper body wearing unit 156a. It is conceivable that an upper body wearing unit comprises no belt and only suspenders, or vice versa. It is also conceivable that a wearable sitting posture assisting device is only connected to the legs and/or the feet and/or the shoes of a person who it is worn by.

The wearable sitting posture assisting device 100a comprises a body connection unit 10a for connecting to the shank 212a of the person 200a. The body connection unit 10a is mounted to the leg unit 102a. In the case shown the body connection unit 10a is mounted to the lower leg 124a, in particular to the lower leg support 126a. The wearable sitting posture assisting device 100a comprises a second body connection unit 12a. The second body connection unit 12a is mounted to the second leg unit 104a. The second body connection unit 12a is implemented identically to the body connection unit 10a, in particular mirror-symmetrically. In the following, only the body connection unit 10a is described in more detail. The exemplary embodiment of the body connection unit 10a as a shank connection unit is to be understood as purely exemplary. It is conceivable that a thigh connection unit is implemented analogously to the shown body connection unit 10a. In particular, where reference is made to the shank 212a of the person 200a for the shown case of a shank connection unit the respective body part may as well be the thigh 208a and/or the knee 202a of the person 200a in case of a body connection unit according to the invention being implemented as thigh connection unit and/or knee connection unit. It is further conceivable that a body connection unit is configured for connecting to a shank and a thigh, in particular to a knee of a person. It is further conceivable that one leg unit comprises more than one body connection units, in particular configured for connecting to different body parts of a person. It is further conceivable that a wearable sitting posture assisting device comprises different leg units and/or that the leg units of a wearable sitting posture assisting device comprises differently implemented body connection units and/or a different number of body connection units.

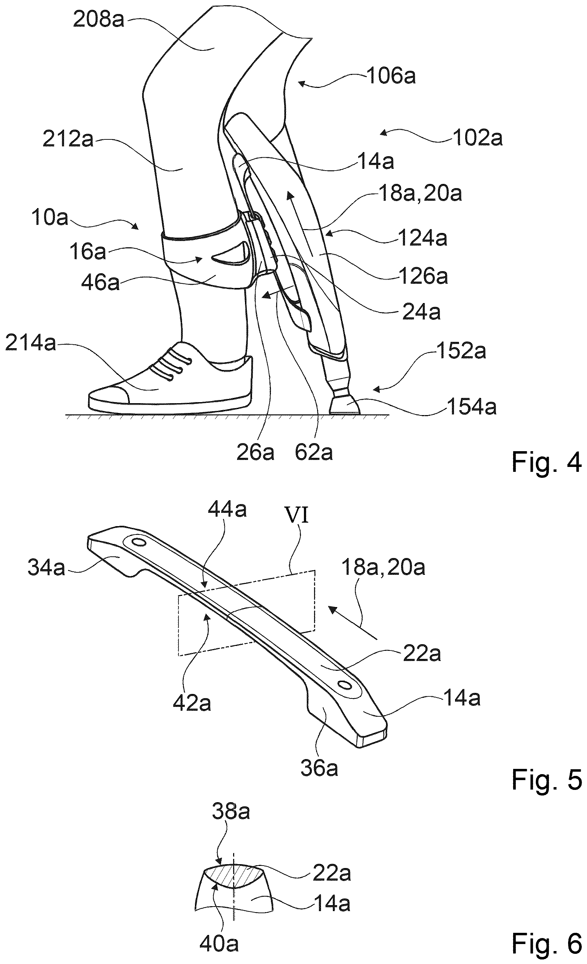

Figure 4 shows a portion of the wearable sitting posture assisting device 100a comprising the body connection unit 10a in a first wearing state, in a schematic lateral view. In the first wearing state the person 200a is sitting on the wearable sitting posture assisting device 100a. The body connection unit 10a comprises a support element 14a. In the case shown the support element 14a is mounted to the lower leg support 126a. Furthermore, the body connection unit 10a comprises a body part connector 16a for connecting to the shank 212a of the person 200a. As mentioned above, it is also conceivable that a body part connector 16a is configured for connecting to the thigh 208a of the person 200a. The body part connector 16a is movably mounted to the support element 14a. It is also conceivable that a support element is at least partly implemented integrally with a lower leg, for instance with a lower leg support. In particular, it is conceivable that a lower leg implements a portion, in particular at least a support element, of a body connection unit.

The body part connector 16a is movable in a direction 18a that is at least substantially parallel to a main extension direction 20a of the support element 14a. In the case shown the main extension direction 20a of the support element 14a is oriented at least substantially parallel to the lower leg longitudinal axis 128a (compare Fig. 1). The body part connector 16a is slidably mounted to the support element 14a. The body part connector 16a is slidable along the support element 14a parallel to the main extension direction 20a of the support element 14a. As a result, a comfortably large free moving space can be achieved, for instance when changing a sitting position, in particular due to a compensation movement of the body part connector 16a along the support element 14a.

Figure 5 shows the support element 14a of the body connection unit 10a, in a perspective view. Figure 6 shows the support element 14a in a schematic sectional view along a section plane VI in figure 5. The support element 14a comprises a rail element 22a. The support element 14a comprises a first connection element 34a and a second connection element 36a for connecting the rail element 22a to the lower leg support 126a. The connection element 34a, 36a and the rail element 22a are at least partly implemented integrally. The rail element 22a comprises a cross section which features a first convex side 38a and a second convex side 40a. The second convex side 40a is more convex than the first convex side 38. The rail element 22a features one more strongly curved rear surface 42a and one less strongly curved front surface 44a. As a result, the body part connector 16a is guided along the rail element 22, in particular in a torque-proof fashion. Furthermore, low wear can be achieved even in case of iterated pulling on and pushing against the rail element 22a of the body part connector 16a. In the case shown the support element 14a features a length along its main extension direction 20a of approximately 30 cm. However, lengths of no more than 20 cm or of no more than 10 cm or of more than 30 cm, for instance between 30 cm and 40 cm or between 40 cm and 50 cm or other lengths are also conceivable, in particular for the support element 14a and/or for the rail element 22a. Furthermore, in the case shown the rail element 22a and/or the support element 14a are made of plastic. However, as mentioned above, it is conceivable that a support element and/or a rail element is at least partly or at least to a large extent made of metal.

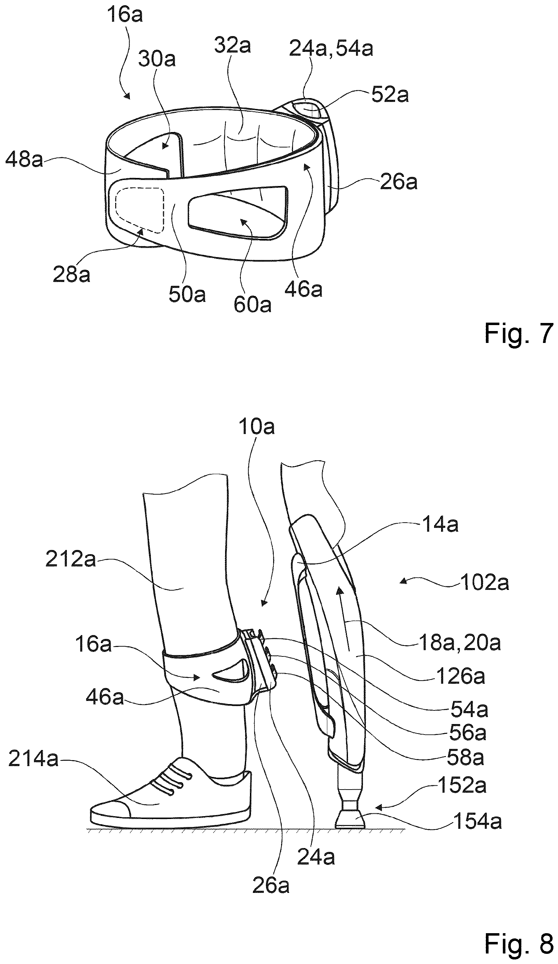

Figure 7 shows the body part connector 16a, in a perspective view. The body part connector 16a comprises a body connection element 46a for connecting to the shank 212a of the person 200a. The body connection element 46a is configured for encompassing the shank 212a of the person 200a in a connected state. The body connection element 46a comprises a first binding element 48a and a second binding element 50a. In the case shown the binding elements 48a, 50a are implemented as at least partly flexible and/or deformable bracket element. In the first wearing state the binding elements 48a, 50a together encompass the shank 212a of the person 200a.

The body connection unit 10a comprises a sliding element 24a which connects the body part connector 16a to the support element 14a. In the case shown the sliding element 24a is part of the body part connector 16a. Furthermore, in the case shown the body connection element 46a implements the sliding element 24a. The sliding element 24a implements a link region 52a for linking to the support element 14a. In a mounted state the support element 14a protrudes through the linking region 52a. The sliding element 24a encompasses the support element 14a. A cross section of the link region 52a corresponds to and/or is, at least within a tolerance of no more than 2 mm, preferably of no more than 1 mm, identical to the cross section of the support element 14a, in particular to a cross section of the rail element 22a.

The body part connector 16a comprises a quick fastener 28a. In the case shown the quick fastener 28a is implemented as a magnetic quick fastener. The binding elements 48a, 50a together implement the quick fastener 28a. A quick fastener may also be implemented as an electromagnetic quick fastener, a click-in quick fastener, a hook-and-loop quick fastener or the like.

The body part connector 16a, in particular the body connection element 46a, comprises at least one ventilation section 30a, 60a, which is at least air-permeable. In the case shown the ventilation section 30a is implemented as an opening. The ventilation section 30a is implemented as an opening in the first binding element 48a. The ventilation section 30a is configured for allowing an air flow to the shank 212a of the person 200a from an environment. In the case shown the body part connector 16a comprises a second ventilation section 60a, which is in particular implemented mirror-symmetrically to the ventilation section 30a. The second ventilation section 60a is implemented as an opening in the second binding element 50a. It is also conceivable that a ventilation section comprises at least one material which is air-permeable.

The body part connector 16a, in particular the body connection element 46a, comprises at least one padding element 32a. In the case shown the padding element 32a is in a wearing state arranged on a rear side of the shank 212a of the person 200a. In the wearing state the padding element 32a is in contact with the shank 212a of the person 200a, in particular with the rear side of the shank 212a of the person 200a. The padding element 32a may comprise a breathable material, in particular as a top layer.

Figure 8 shows the portion of the wearable sitting posture assisting device 100a in a disconnected state of the body connection unit 10a, in a schematic lateral view. The body part connector 16a is connectable to the support element 14a. The body part connector 16a is disconnectable from the support element 14a. The body part connector 16a is connectable to and/or disconnectable from the support element 14a without tools. In the case shown the body part connector 16a comprises a fastening element 54a for fastening to the support element 14a. In a mounted state of the body part connector 16a the fastening element 54a inhibits removal of the body part connector 16a from the support element 14a. However, the body part connector 16a is in the mounted state movably mounted to the support element 14a. In the case shown, the fastening element 54a is a part of the sliding element 24a. In particular, the fastening element 54a implements at least a portion of a rear side of the sliding element 24a. The fastening element 54a may be spring-loaded. In particular, the fastening element 54a may snap in and/or out of a fastening position and/or an unfastening position. In the case shown the body part connector 16a, in particular the sliding element 24a, comprises three fastening elements 54a, 56a, 58a. The sliding element 24a, in particular the link region 52a, can be opened and closed using the fastening elements 54a, 56a, 58a for connecting to and/or for disconnecting from the support element 24a.

In a method for putting on or taking off the wearable sitting posture assisting device 100a, the body part connector 16a is connected to or disconnected from the shank 212a of the person 200a in a disconnected state, in which the body part connector 16a is in particular disconnected from the support element 14a. In particular, when putting on the wearable sitting posture assisting device 100a, the body part connector 16a is connected to the shank 212a of the person 200a prior to connecting of the body part connector 16a to the support element 14a, in particular using the fastening elements 54a, 56a, 58a. Preferably, when taking off the wearable sitting posture assisting device 100a, the body part connector 16a is disconnected from the support element 14a prior to disconnecting of the body part connector 16a from the shank 212a of the person 200a. As a result, the person 200a is enabled to put on or take off the body part connector 16a unobstructed by the leg unit 102a.

The body connection unit 10a comprises a reversibly deformable compensation element 26a, which is configured for generating at least one restoring force for the body part connector 16a in at least one deflected state of the body part connector 16a. The compensation element 26a is configured for allowing a movement of the shank 212a of the person 200a relative to the support element 14a in a mounted and wearing state of the body part connector 10a in a direction 62a that is perpendicular to the main extension direction 20a of the support element 14a and in particular perpendicular to a surface normal of a plane defined by the main extension direction 20a of the support element 14a and the sitting direction 134a. As a result, the person 200a can move his foot and/or shoe 214a away from or towards the ground contact element 154a while sitting on the wearable sitting posture assisting device 100a. Furthermore, in the case shown the compensation element 26a is configured for allowing a twisting movement of the shank 212a, in particular over an angle range of approximately 20°, in a mounted state of the body part connector 16a. In the case shown the compensation element 26a permanently connects the sliding element 24a to the body connection element 46a. Furthermore, in the case shown the compensation element 26a is at least partly made of rubber. In particular, the compensation element 26a is a rubber element. It is also conceivable that a compensation element comprises at least one spring and/or at least one plastic material and/or at least one mesh structure and/or at least one porous structure and/or at least one compensation recess and/or compensation opening.

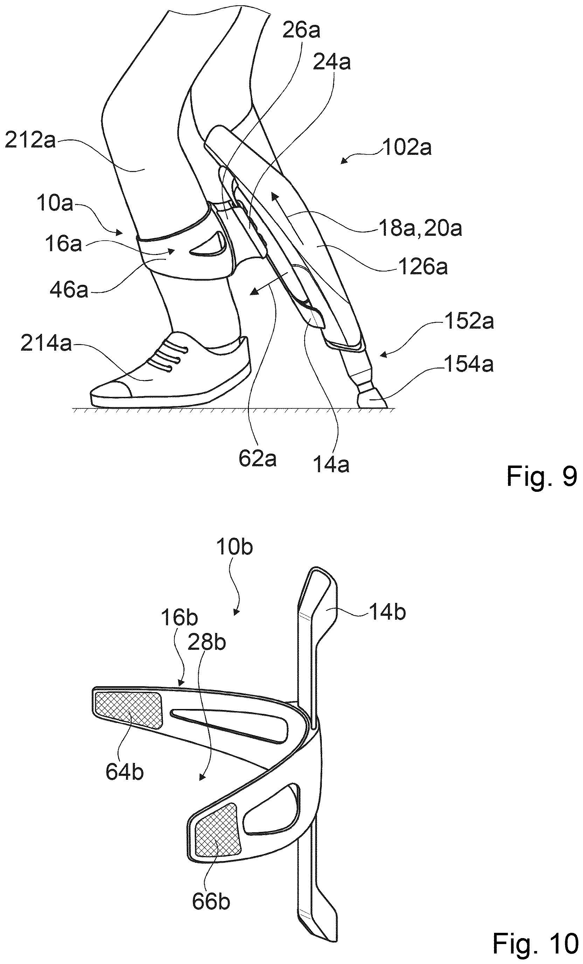

Figure 9 shows the portion of the wearable sitting posture assisting device 100a in a second wearing state, in a schematic lateral view. In the second wearing state the foot and/or shoe 214a of the person 212a is farther away from the ground contact element 154a than in the first wearing state. Furthermore, in the second wearing state the person 200a exerts a pulling force, in particular by his shank 212a, onto the support element 14a, in particular via the body connection element 46a and/or the compensation element 26a and/or the sliding element 24a. The second wearing state may for instance correspond to a different sitting position or the same sitting position as the first wearing state. In the second wearing state the body part connector 16a is in a deflected state, in particular due to the pulling force. In the deflected state, an extension of the body part connector 16a in the direction 62a that is perpendicular to the main extension direction 20a of the support element 14a differs from an extension of the body part connector 16a in this direction 62a in a non-deflected and/or basic state of the body part connector 16a. In the case shown the extension of the body part connector 16a is increased in the second wearing state, in particular due to the pulling force. However, in an analogous manner a compression of the body part connector 16a is also conceivable. The compensation element 26a exerts the restoring force onto the body connection element 46a due to its deformation, which deformation is in particular a result of a deflection force exerted by the shank 212a of the person 200a onto the body connection unit 10a.

Figs. 10 to 14 show further exemplary embodiments of the invention. The following description is substantially limited to the differences between the exemplary embodiments, wherein regarding structural elements, features and functions that remain the same the description of the other exemplary embodiments, in particular the exemplary embodiment of figs. 1 to 9, may be referred to. For distinguishing the exemplary embodiments, the letter a of the reference numerals in the exemplary embodiment of figs. 1 to 9 has been substituted by the letters b to d in the reference numerals of the exemplary embodiments of figs. 10 to 14. Regarding structural elements having the same denomination, in particular regarding structural elements having the same reference numerals, principally the drawing and/or the description of the other exemplary embodiments, in particular of the exemplary embodiment of figs. 1 to 9, may be referred to.

Figure 10 shows a first alternative body connection unit 10b, in a perspective view. The first alternative body connection unit 10b comprises a support element 14b and a body part connector 16b for connecting to a thigh and/or a shank of a person. The body part connector 16b is movably mounted to the support element 14b. For instance, the first alternative body connection unit 10b may be implemented as a thigh connection unit.

The body part connector 16b is permanently connected to the support element 14b. The body part connector 16b is permanently slidably mounted to the support element 14b. The body part connector 16b comprises a sliding element 24b, which permanently connects the body part connector 16b to the support element 14b.

The body part connector 16b comprises a quick fastener 28b. The quick fastener 28b is implemented as a hook-and-loop quick fastener. The quick fastener 28b comprises a first fastening section 64b and a second fastening section 66b.

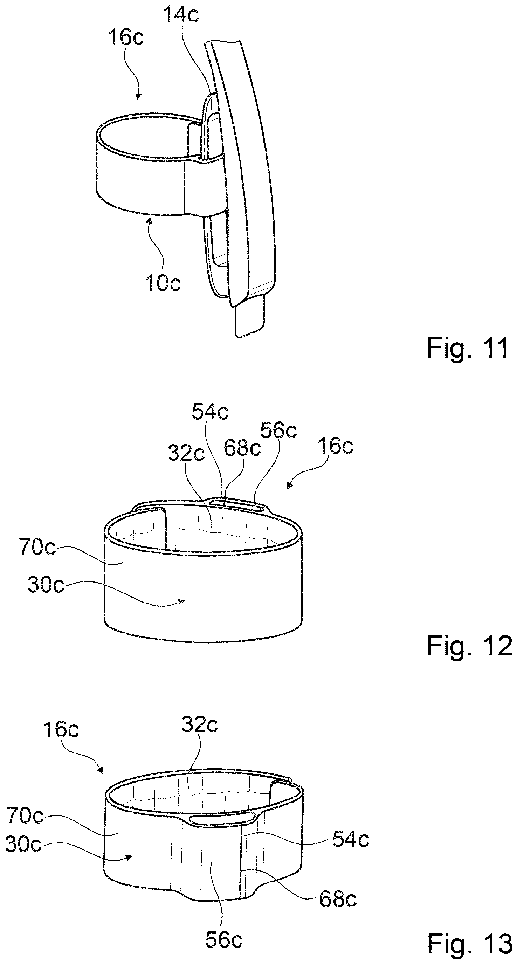

Figure 11 shows a second alternative body connection unit 10c in a perspective view. The second alternative body connection unit 10c comprises a support element 14c and a body part connector 16c for connecting to a thigh and/or a shank of a person. The body part connector 10c is movably mounted to the support element 14c.

Figure 12 shows the body part connector 16c in a perspective front view. Figure 13 shows the body part connector 16c in a perspective rear view. The body part connector 16c is slidably mounted to the support element 14c. The body part connector 16c is connectable to and disconnectable from the support element 14c. The body part connector 16c comprises two fastening elements 54c, 56c for mounting to the support element 14c. The fastening elements 54c, 56c are arranged opposite each other with respect to an opening slit 68c, which can be opened and closed for connecting and disconnecting the body part connector 16c. The fastening elements 54c, 56c implement a quick connector, which opens and closes at the opening slit 68c.

The body part connector 16c comprises a strap element 70c for connecting to the thigh and/or shank. The strap element 70c comprises a padding element 32c, which extends around the thigh or shank in a wearing state. The strap element 70c is made of a breathable material. The strap element 70c is at least partly air-permeable. The strap element 70c implements a ventilation section 30c.

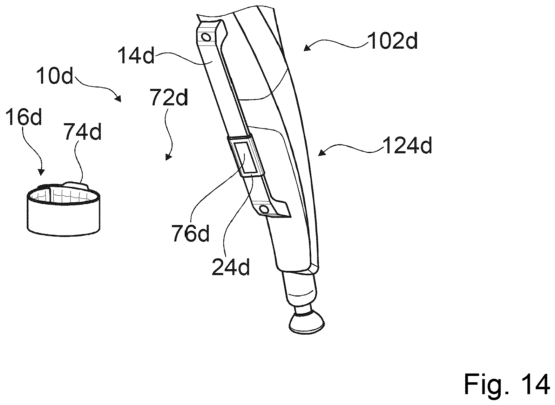

Figure 14 shows a third alternative body connection unit 10d, in a perspective view. The third alternative body connection unit 10d is implemented as a shank connection unit. The third alternative body connection unit 10d is mounted to a lower leg 124d of a leg unit 102d of an alternative wearable sitting posture assisting device. It is of course also conceivable that the third alternative body connection unit 10d is implemented as a thigh connection unit.

The third alternative body connection unit 10d comprises a support element 14d and a body part connector 16d for connecting to a shank of a person. The body part connector 16d is in a mounted state movably mounted to the support element 14d. The body part connector 16d is connectable to and/or disconnectable from the support element 14d. In figure 14 the body part connector 16d is shown in a disconnected state. The body connection unit 10d comprises a sliding element 24d, which connects the body part connector 16d to the support element 16d in the mounted state. The sliding element 24d is permanently slidably mounted to the support element 14d. The body part connector 16d is connectable to and/or disconnectable from the sliding element 24d. The body part connector 16d and the sliding element 24d together implement a quick connector 72d for the connection of the body part connector 16d to the sliding element 24d. The quick connector 72d comprises a first connection element 74d of the body part connector 16d, which is connectable to and/or disconnectable from a second connection element 76d of the sliding element 24d. In the case shown the quick connector 72d is implemented as a hook-and-loop quick fastener. It is also conceivable that a respective quick connector is implemented as a magnetic quick connector and/or as an electromagnetic quick connector and/or as a click-in connector and/or as a snap connector or the like.

## Claims

1. A wearable sitting posture assisting device, comprising at least one body connection unit (10a, 12a; 10b-d), the body connection unit (10a, 12a; 10b-d) featuring at least one support element (14a-d) and at least one body part connector (16a-d) for connecting to at least one thigh (208a) and/or shank (212a) of a person (200a), **characterized in that** the body part connector (16a-d) is movably mounted to the support element (14a-d).

2. The wearable sitting posture assisting device according to claim 1, **characterized in that** the body part connector (16a-d) is movable in a direction (18a) that is at least substantially parallel to a main extension direction (20a) of the support element (14a-d).

3. The wearable sitting posture assisting device according to any one of claims 1 or 2, **characterized in that** the body part connector (16a-d) is slidably mounted to the support element (14a-d).

4. The wearable sitting posture assisting device according to any one of the preceding claims, **characterized in that** the support element (14a-d) comprises at least one rail element (22a).

5. The wearable sitting posture assisting device according to any one of the preceding claims, **characterized in that** the body connection unit (10a, 12a; 10b-d) comprises at least one sliding element (24a; 24b; 24d) which connects the body part connector (16a-d) to the support element (14a-d).

6. The wearable sitting posture assisting device according to any one of the preceding claims, **characterized in that** the body connection unit (10a, 12a) comprises at least one reversibly deformable compensation element (26a) which is configured for generating at least one restoring force for the body part connector (16a) in at least one deflected state of the body part connector (16a).

7. The wearable sitting posture assisting device according to any one of the preceding claims, **characterized in that** the body part connector (16a; 16c; 16d) is connectable to and/or disconnectable from the support element (14a; 14c; 14d).

8. The wearable sitting posture assisting device according to any one of the preceding claims, **characterized in that** the body part connector (16a-d) comprises at least one quick fastener (28a; 28b).

9. The wearable sitting posture assisting device according to any one of the preceding claims, **characterized in that** the body part connector (16a-d) comprises at least one ventilation section (30a, 60a; 30c), which is at least partially air-permeable.

10. The wearable sitting posture assisting device according to any one of the preceding claims, **characterized in that** the body part connector (16a-d) comprises at least one padding element (32a; 32c).

11. The wearable sitting posture assisting device according to any one of the preceding claims, **characterized in that** the body part connector (16a-d) is implemented as a shank connector.

12. The wearable sitting posture assisting device according to any one of the preceding claims, **characterized by** at least one leg unit (102a, 104a) to which the body connection unit (10a-d) is mounted.

13. The wearable sitting posture assisting device according to claim 12, **characterized in that** the leg unit (102a) comprises at least one upper leg (106a) and at least one lower leg (124a), to which lower leg (124a) the body connection unit (10a-d) is mounted.

14. The wearable sitting posture assisting device according to any one of claims 12 or 13, **characterized by** a second leg unit (104a), which is implemented at least substantially identically to the leg unit (102a).

15. A method of putting on or taking off a wearable sitting posture assisting device (100a) according to any one of the preceding claims, wherein the body part connector (10a; 10c; 10d) is connected to or disconnected from the thigh (208a) and/or the shank (212a) of the person (200a) in a disconnected state.
